# EUROPÄISCHE PATENTSCHRIFT

(11) **EP 3 570 759 B1**
(45) Veröffentlichungstag und Bekanntmachung des Hinweises auf die Patenterteilung: **23.08.2023**
(21) Anmeldenummer: 18701709.0
(22) Anmeldetag: 22.01.2018
(51) Int. Cl.: A61B 17/15, A61B 17/16

(54) **MEDIZINISCHES INSTRUMENTARIUM MIT SÄGESCHABLONE**
MEDICAL APPARATUS COMPRISING SAWING TEMPLATE
INSTRUMENT MÉDICAL AVEC GABARIT DE SCIAGE

(30) Priorität: 20.01.2017 DE 102017101135
(43) Veröffentlichungstag der Anmeldung: 27.11.2019
(73) Patentinhaber: Aesculap AG, 78532 Tuttlingen (DE)
(72) Erfinder: BADER, Uwe, 78532 Tuttlingen (DE); VARSANI, Anil, 78532 Tuttlingen/Möhringen (DE); STOLL, Stephanie, 78662 Herrenzimmern (DE)
(74) Vertreter: Hoeger, Stellrecht & Partner Patentanwälte mbB
(86) Internationale Anmeldenummer: PCT/EP2018/051371
(87) Internationale Veröffentlichungsnummer: WO 2018/134383

(56) Entgegenhaltungen:
- FR-A1- 2 854 786
- US-B1- 6 206 884

## Beschreibung

Die vorliegende Erfindung betrifft ein medizinisches Instrumentarium zum Implantieren einer Hüftgelenkendoprothese, welches Instrumentarium mindestens eine Sägeschablone mit mindestens einem Führungsschlitz für ein Sägeblatt umfasst, wobei die Sägeschablone mindestens ein erstes Kopplungselement zum temporären Koppeln der Sägeschablone mit einem zweiten Kopplungselement eines Raspelkörpers einer Prothesenraspel in einer Kopplungsstellung umfasst, wobei dem ersten Kopplungselement ein erstes Sicherungselement einer Sicherungseinrichtung zugeordnet ist, welches in der Kopplungsstellung mit einem am Raspelkörper angeordneten zweiten Sicherungselement in einer Sicherungsstellung kraft- und/oder formschlüssig in Eingriff steht.

Geschädigte Hüftgelenke werden seit vielen Jahren durch Hüftgelenkendoprothesen ersetzt. Einen Teil solcher Hüftgelenkendoprothesen bildet ein Prothesenschaft, der in eine dafür vorbereitete Femurkavität eingesetzt wird. Am Prothesenschaft ist eine Gelenkkugel angeordnet, die zusammen mit einer im Becken des Patienten verankerten Gelenkpfanne ein Kugelgelenk ausbildet.

Prothesenschäfte von Hüftgelenkendoprothesen werden insbesondere zementfrei im Femur verankert. Dabei ist es wünschenswert, wenn der Femur im Bereich des Femurhalses so reseziert wird, dass er optimal mit einem aus dem Femurknochen herausragenden Ende des Prothesenschafts abschließt.

Eine solche Resektion kann beispielsweise mit einer Sägeschablone erfolgen. Da diese jedoch keine fest vorgegebene Referenz am Femur haben, kommt es bei deren Einsatz üblicherweise zu größeren Winkelfehlern und einem Versatz der Resektionshöhe. Mit anderen Worten wird zu viel oder zu wenig vom Femurknochen abgenommen, sodass ein proximales Ende des Femurs nicht wie gewünscht im proximalen Endbereich des Prothesenschafts endet.

Es ist daher eine Aufgabe der vorliegenden Erfindung, ein medizinisches Instrumentarium der eingangs beschriebenen Art so zu verbessern, dass Prothesenschäfte optimal im Femurknochen verankert werden können.

Aus der US 6,206,884 B1 ist eine Vorrichtung zum Ersetzen von Gelenken bekannt. Eine Vorrichtung zum Präparieren eines ebenen Femurschnitts ist in der FR 2 854 786 A1 beschrieben.

Diese Aufgabe wird bei einem medizinischen Instrumentarium der eingangs beschriebenen Art erfindungsgemäß dadurch gelöst, dass das erste und das zweite Sicherungselement in Form von zusammenwirkenden Rastgliedern ausgebildet sind.

Die erfindungsgemäß vorgeschlagene Lösung ermöglicht es insbesondere auf einfache Weise, die Sägeschablone beispielsweise in definierter Weise mit einem Raspelkörper einer Prothesenraspel in der Kopplungsstellung zu koppeln. Eine Prothesenraspel wird zum Vorbereiten der Knochenkavität am Femurknochen des Patienten genutzt, um die Knochenkavität so zu präparieren, dass der endgültig zu implantierende Prothesenschaft perfekt in der Femurkavität sitzt. Dies ist insbesondere dann wichtig, wenn der Prothesenschaft zementfrei verankert wird. Der Prothesenschaft kann insbesondere eine osteointegrative Beschichtung aufweisen, die das Einwachsen von Knochen erleichtert. So kann der nur durch einen Klemmsitz in der Knochenkavität gehaltene Prothesenschaft dauerhaft sicher mit dem Femurknochen verbunden werden. Da der Raspelkörper der Prothesenraspel mit seiner Außenkontur dem zu implantierenden Prothesenschaft entspricht, ist somit auch die endgültige Position des Prothesenschafts im Femurknochen bekannt, und zwar durch den in die Femurkavität eingeschlagenen Raspelkörper. Dieser kann dann relativ zum Femurknochen eine Referenz für die Sägeschablone bilden. Das zweite Kopplungselement an der Sägeschablone ermöglicht so insbesondere eine definierte Verbindung mit dem Raspelkörper, sodass der Femurknochen durch Einsatz der Sägeschablone in gewünschter Weise teilreseziert werden kann, und zwar entsprechend einem proximalen Ende des zu implantierenden Prothesenschafts oder einer proximalen Endlinie der osteointegrativen Beschichtung am zu implantierenden Prothesenschaft. Hierfür kann ein Sägeblatt durch den mindestens einen Führungsschlitz der Sägeschablone, welche mit dem Raspelkörper der Prothesenraspel gekoppelt ist, in definierter Weise geführt werden. Gemäß der Erfindung ist vorgesehen, dass dem ersten Kopplungselement ein erstes Sicherungselement einer Sicherungseinrichtung zugeordnet ist, welches in der Kopplungsstellung mit einem am Raspelkörper angeordneten zweiten Sicherungselement in einer Sicherungsstellung kraft- und/oder formschlüssig in Eingriff steht. Insbesondere kann das erste Sicherungselement derart angeordnet sein, dass es in einer Richtung wirkt, die quer zur Kopplungsrichtung verläuft, also quer zur Richtung, in der die ersten und zweiten Kopplungselemente miteinander in Eingriff gebracht werden können. So kann auf einfache Weise verhindert werden, dass sich die Sägeschablone und der Raspelkörper in nicht gewünschter Weise voneinander lösen können. Auf einfache Weise lassen sich die Sägeschablone und der Raspelkörper in der Kopplungsstellung aneinander sichern, weil das erste und das zweite Sicherungselement in Form von zusammenwirkenden Rastgliedern ausgebildet sind. Diese können beim in Eingriff Bringen der ersten und zweiten Kopplungselemente beispielsweise automatisch ineinandergreifen und verrasten zum Sichern einer Verbindung zwischen der Sägeschablone und dem Raspelkörper.

Günstig ist es, wenn die Sägeschablone einen Kopplungskörper und einen Schablonenkörper umfasst und wenn das mindestens eine erste Kopplungselement am Kopplungskörper angeordnet oder ausgebildet ist und wenn der mindestens eine Führungsschlitz am Schablonenkörper angeordnet oder ausgebildet ist. Insbesondere können der Kopplungskörper und der Schablonenkörper einstückig ausgebildet oder dauerhaft miteinander verbunden sein, beispielsweise durch Kleben, Schweißen, Löten oder dergleichen.

Vorteilhaft ist es, wenn das Instrumentarium eine Kupplungseinrichtung zum temporären Kuppeln des Kopplungskörpers und des Schablonenkörpers in einer Kupplungsstellung umfasst. Die Kupplungseinrichtung ermöglicht es insbesondere, den Kopplungskörper und den Schablonenkörper temporär kraft- und/oder formschlüssig miteinander in der Kupplungsstellung zu kuppeln. So lässt sich der Schablonenkörper vom Kopplungskörper auf einfache Weise trennen und bei Bedarf wieder mit diesem verbinden. So kann insbesondere eine Reinigbarkeit des Instrumentariums verbessert werden. Zudem kann das Instrumentarium optional auch modular ausgebildet werden. Beispielsweise können unterschiedliche Schablonenkörper mit demselben Kopplungskörper in Eingriff gebracht werden, um den Femurknochen des Patienten teilweise zu resezieren. Dabei kann insbesondere unterschiedlichen Raspelkörpern jeweils ein spezifischer Schablonenkörper zugeordnet werden, um so eine definierte Referenz für einen Operateur zum Bearbeiten des Femurs vorgeben zu können.

Auf einfache Weise lassen sich der Kopplungskörper und der Schablonenkörper kuppeln, wenn die Kupplungseinrichtung in Form einer Rast- oder Schnappverbindungseinrichtung ausgebildet ist und in der Kupplungsstellung miteinander in Eingriff stehende Rast- oder Schnappelemente umfasst.

Günstig ist es, wenn die Kupplungseinrichtung erste und zweite Kupplungselemente umfasst, wenn die ersten und zweiten Kupplungselemente einerseits am Kopplungskörper und andererseits am Schablonenkörper angeordnet oder ausgebildet sind und wenn die ersten und zweiten Kupplungselemente in der Kupplungsstellung kraft- und/oder formschlüssig in Eingriff stehen. Durch das in Eingriff Bringen der ersten und zweiten Kupplungselemente lassen sich also der Kopplungskörper und der Schablonenkörper auf einfache Weise temporär miteinander verbinden.

Vorteilhafterweise bilden die ersten und zweiten Kupplungselemente die Rast- oder Schnappelemente. So lässt sich die Kupplungseinrichtung auf einfache Weise ausbilden.

Gemäß einer weiteren bevorzugten Ausführungsform der Erfindung kann vorgesehen sein, dass der Kopplungskörper und der Schablonenkörper in der Kupplungsstellung relativ zueinander um eine durch die Kupplungseinrichtung definierte Drehachse verdrehbar aneinander gelagert sind. Diese Ausgestaltung ermöglicht es insbesondere, den Kopplungskörper und den Schablonenkörper zwar definiert zu kuppeln, beispielsweise in einer durch die Drehachse vorgegebenen Richtung. Gleichzeitig kann jedoch noch eine Verdrehung des Kopplungskörpers und des Schablonenkörpers relativ zueinander ermöglicht werden. So kann der Schablonenkörper insbesondere in gewünschter Weise ausgerichtet werden, um den Femurknochen des Patienten gezielt zu bearbeiten. Eine solche Verdrehmöglichkeit gestattet es einem Operateur, den Femurknochen aus unterschiedlichen Richtungen zu bearbeiten. So kann insbesondere der Schablonenkörper sehr klein und kompakt ausgebildet werden.

Die Ausbildung der Kupplungseinrichtung vereinfacht sich weiter, wenn die ersten und zweiten Kupplungselemente in Form mindestens eines Kupplungsvorsprungs und in Form einer korrespondierenden Kupplungsausnehmung ausgebildet sind. Zum Kuppeln des Schablonenkörpers und des Kopplungskörpers kann so der mindestens eine Kupplungsvorsprung in die korrespondierende Kupplungsausnehmung eingeführt werden.

Vorzugsweise ist der mindestens eine Kupplungsvorsprung federnd am Schablonenkörper angeordnet oder ausgebildet. Alternativ kann er auch am Kopplungskörper angeordnet oder ausgebildet sein. Die federnde Ausgestaltung ermöglicht es insbesondere, den Kupplungsvorsprung in die korrespondierende Kupplungsausnehmung einschnappen zu lassen.

Günstig ist es, wenn der mindestens eine Kupplungsvorsprung in Form eines auf die Drehachse hin weisend konkav gekrümmten Haltebügels ausgebildet ist. Insbesondere können zwei oder mehr derartige Kupplungsvorsprünge vorgesehen sein, die in eine von der Drehachse weg weisende und diese, insbesondere konzentrisch, umgebende Ringnut am Kopplungskörper eingreifen.

Um eine sichere Verbindung zwischen dem Schablonenkörper und dem Kopplungskörper zu erreichen, ist es vorteilhaft, wenn der mindestens eine Kupplungsvorsprung in Form eines in sich geschlossenen oder sich über mindestens 180° in Umfangsrichtung bezogen auf die Drehachse erstreckenden Kupplungsrings ausgebildet ist. Auf diese Weise lassen sich der Kopplungskörper und der Schablonenkörper einfach und sicher kuppeln. Zudem kann so optional auch auf einfache Weise eine Verdrehung des Kopplungskörpers und des Schablonenkörpers relativ zueinander um die Drehachse ermöglicht werden.

Besonders kompakt ausbilden lässt sich das Instrumentarium, wenn die mindestens eine Kupplungsausnehmung in Form einer von der Drehachse weg weisend geöffneten Ringnut ausgebildet ist und wenn der mindestens eine Kupplungsvorsprung in der Kupplungsstellung in Umfangsrichtung bezogen auf die Drehachse mindestens abschnittsweise in die Kupplungsausnehmung eingreift. Insbesondere können zwei oder mehr Kupplungsvorsprünge vorgesehen sein, die in der Kupplungsstellung in die Ringnut eingreifen. Insbesondere lassen sich Kupplungsvorsprünge in einer Form ausbilden, welche es ermöglicht, dass die Kupplungsvorsprünge in die Ringnut einschnappen oder einrasten können.

Vorteilhaft ist es, wenn die Ringnut seitlich von einem Haltering begrenzt ist. So lässt sich die Ringnut zum einen auf einfache Weise herstellen. Zum anderen kann optional mit dem Haltering auch ein in sich geschlossener Kupplungsring in der Ringnut gesichert werden. Der Haltering kann optional dauerhaft oder temporär an der Kupplungsausnehmung angeordnet oder ausgebildet sein.

Günstig ist es, wenn der Haltering und der Kopplungskörper kraft- und/oder form- und/oder stoffschlüssig miteinander verbunden sind. So kann insbesondere der Schablonenkörper dauerhaft mit dem Kopplungskörper gekoppelt bleiben.

Wie oben beschrieben ist es optional möglich, den Kopplungskörper und den Schablonenkörper relativ zueinander um die Drehachse zu verdrehen. Dabei kann es insbesondere günstig sein, wenn die ersten und zweiten Kupplungselemente relativ zueinander in diskreten Winkelstellungen bezogen auf die Drehachse ausrichtbar sind. Insbesondere kann hier eine Mehrzahl von diskreten Winkelstellungen vorgesehen sein. So lässt sich insbesondere der Schablonenkörper relativ zum Kopplungskörper in gewünschter Weise ausrichten.

Gemäß einer weiteren bevorzugten Ausführungsform der Erfindung kann vorgesehen sein, dass das Instrumentarium eine Winkeleinstelleinrichtung zum Einstellen einer Winkelstellung des Kopplungskörpers und des Schablonenkörpers bezogen auf die Drehachse relativ zueinander umfasst. Die Winkeleinstelleinrichtung ermöglicht es insbesondere, den Schablonenkörper und den Kopplungskörper relativ zueinander bezogen auf die Drehachse in gewünschter Weise auszurichten.

Günstig ist es, wenn die Winkeleinstelleinrichtung eine Mehrzahl erster Winkeleinstellglieder und mindestens ein zweites Winkeleinstellglied umfasst und wenn das mindestens eine zweite Winkeleinstellglied in unterschiedlichen Winkelstellungen mit unterschiedlichen ersten Winkeleinstellgliedern in Eingriff steht. Beispielsweise kann das mindestens eine zweite Winkeleinstellglied ausgebildet sein, um in unterschiedlichen Winkelstellungen mit der Mehrzahl erster Winkeleinstellglieder rastend in Eingriff zu kommen.

Auf einfache Weise ausbilden lässt sich die Winkeleinstelleinrichtung, wenn die Mehrzahl erster Winkeleinstellglieder in Form von Winkelgliedausnehmungen ausgebildet sind und wenn das mindestens eine zweite Winkeleinstellglied in Form eines Winkelgliedvorsprungs ausgebildet ist. Die Mehrzahl erster Winkeleinstellglieder kann so insbesondere in Form einer Zahnreihe ausgebildet werden, wobei das mindestens eine zweite Winkeleinstellglied in unterschiedlichen Winkelstellungen zwischen benachbarte Zähne der Zahnreihe eintauchen kann.

Vorteilhaft ist es, wenn die Winkelgliedausnehmungen am ersten oder zweiten Kupplungselement angeordnet oder ausgebildet sind und in Richtung auf die Drehachse hin oder von der Drehachse weg weisend angeordnet oder ausgebildet sind. So lässt sich der Schablonenkörper auf einfache Weise relativ zum Kopplungskörper in definierten Winkelstellungen ausrichten.

Günstig ist es, wenn der mindestens eine Winkelgliedvorsprung federnd gelagert ist und beim Ändern der Winkelstellung durch Drehen der ersten und zweiten Kupplungselemente relativ zueinander um die Drehachse entgegen einer vorspannenden Kraft aus einer der Winkelgliedausnehmungen hinaus bewegt und in einer anderen Winkelstellung durch die vorspannende Kraft in eine der Winkelgliedausnehmungen zurück bewegt wird. Diese Ausgestaltung ermöglicht es einem Operateur, durch einfaches Verdrehen des Schablonenkörpers und des Kopplungskörpers relativ zueinander, eine durch die Winkeleinstellausrichtung definierte Winkelstellung zwischen dem Schablonenkörper und dem Kopplungskörper zu ändern.

Vorteilhaft ist es, wenn der mindestens eine Führungsschlitz eine Führungsebene definiert, welche quer zur Drehachse verläuft. Insbesondere kann die Führungsebene senkrecht zur Drehachse verlaufen. Optional kann der Schablonenkörper zwei, drei oder mehr Führungsschlitze aufweisen, die unterschiedlichen Prothesenschäften zugeordnet sind. So kann ein Operateur mit einem einzigen Schablonenkörper einen Femurknochen in Abhängigkeit des zu implantierenden Prothesenschafts in gewünschter Weise resezieren. Auf diese Weise lässt sich die Zahl der erforderlichen Schablonenkörper deutlich minimieren.

Auf einfache Weise handhaben lässt sich das Instrumentarium, wenn der Kopplungskörper einen Griffbereich definiert. An diesem lässt sich der Kopplungskörper vom Operateur greifen und so beispielsweise mit dem Raspelkörper in Eingriff bringen.

Einfach und kostengünstig ausbilden lässt sich das Instrumentarium beispielsweise dadurch, dass der Griffbereich symmetrisch bezogen auf die Drehachse ausgebildet ist. Insbesondere kann er rotationssymmetrisch bezogen auf die Drehachse ausgebildet sein.

Gemäß einer weiteren bevorzugten Ausführungsform der Erfindung kann vorgesehen sein, dass das medizinische Instrumentarium einen Raspelkörper einer Prothesenraspel umfasst, welcher Raspelkörper ein zweites Kopplungselement einer das erste und das zweite Kopplungselement umfassenden Kopplungseinrichtung umfasst. Insbesondere können das erste und das zweite Kopplungselement korrespondierend zueinander ausgebildet sein, um den Schablonenkörper und den Kopplungskörper in eindeutiger und definierter Weise miteinander zu koppeln. Insbesondere kann abhängig von einer Ausgestaltung der Kopplungselemente eine axiale und/oder drehfeste Kopplung ermöglicht werden.

Günstig ist es, wenn die ersten und zweiten Kopplungselemente in der Kopplungsstellung kraft- und/oder formschlüssig in Eingriff stehen und wenn sie in einer Reinigungsstellung vollständig voneinander getrennt sind. So lassen sich der Schablonenkörper und der Kopplungskörper auf einfache Weise miteinander in Eingriff bringen und wieder voneinander trennen, insbesondere zu Reinigungszwecken.

Auf einfache Weise ausbilden lässt sich das Instrumentarium, wenn das erste Kopplungselement in Form einer Kopplungselementaufnahme ausgebildet ist. Insbesondere kann das erste Kopplungselement am Kopplungskörper angeordnet oder ausgebildet sein.

Günstig ist es, wenn das zweite Kopplungselement in Form eines zur Kopplungselementaufnahme korrespondierenden Kopplungsvorsprungs ausgebildet ist. Ein solcher Kopplungsvorsprung kann insbesondere auf einfache Weise hergestellt werden.

Die Kopplung zwischen der Sägeschablone und dem Raspelkörper lässt sich insbesondere weiter vereinfachen, wenn die Kopplungseinrichtung eine Längsachse definiert, welche insbesondere vom ersten und/oder zweiten Kopplungselement definiert ist. Beispielsweise kann das zweite Kopplungselement in Form eines Kopplungsvorsprungs ausgebildet sein, welcher die Kopplungslängsachse definiert. Der Kopplungsvorsprung kann insbesondere rotationssymmetrisch bezogen auf die Kopplungslängsachse und/oder die Drehachse ausgebildet sein.

Besonders einfach und kompakt ausbilden lässt sich das Instrumentarium, wenn die Kopplungslängsachse parallel oder im Wesentlichen parallel zur Drehachse verläuft.

Vorzugsweise definiert die Drehachse die Kopplungslängsachse. Diese Ausgestaltung erleichtert insbesondere die Handhabung des Instrumentariums für einen Operateur.

Vorteilhaft ist es, wenn die ersten und zweiten Kopplungselemente in der Kopplungsstellung drehfest miteinander gekoppelt sind. So kann eine sichere und optional auch spielfreie Verbindung zwischen der Sägeschablone und dem Raspelkörper erreicht werden.

Günstig ist es, wenn eines der beiden Rastglieder in Form eines Rastvorsprungs ausgebildet ist und wenn das andere der beiden Rastglieder in Form einer korrespondierenden Rastausnehmung ausgebildet ist. So lässt sich eine Rastverbindungseinrichtung auf einfache Weise ausbilden zum Sichern der in der Kopplungsstellung miteinander in Eingriff stehenden ersten und zweiten Kopplungselemente.

Vorteilhaft ist es, wenn eines der beiden Sicherungselemente am ersten Kopplungselement angeordnet oder ausgebildet ist und wenn das andere der beiden Sicherungselemente am zweiten Kopplungselement angeordnet oder ausgebildet ist. So lässt sich das Instrumentarium einfach und kompakt ausbilden.

Günstig ist es, wenn der Rastvorsprung derart ausgebildet ist, dass er beim in Eingriff Bringen des ersten Kopplungselements und des zweiten Kopplungselements von einer Grundstellung in eine ausgelenkte Stellung ausgelenkt wird und erst dann, wenn die Kopplungseinrichtung die Kopplungsstellung einnimmt, von der ausgelenkten Stellung in die Rastausnehmung eingreifen kann, so dass die Sicherungseinrichtung die Sicherungsstellung einnimmt. So können die ersten und zweiten Sicherungselemente miteinander verrasten, wenn die ersten und zweiten Kopplungselemente miteinander in Eingriff gebracht werden.

Vorteilhaft ist es, wenn die Sägeschablone zwei, drei, vier oder mehr Führungsschlitze umfasst. Dies ermöglicht es insbesondere, zum Präparieren des Femurknochens einen einzigen Raspelkörper zu nutzen, mit dem die Knochenkavität für unterschiedliche Prothesenschäfte präpariert werden kann. Da die unterschiedlichen Prothesenschäfte auch unterschiedlich in der präparierten Kavität des Femurs sitzen, kann jedem Prothesenschaft ein definierter und gegebenenfalls auch entsprechend gekennzeichneter Führungsschlitz zugeordnet sein. Auf diese Weise lässt sich eine Anzahl erforderlicher Teile des Instrumentariums minimieren. Somit kann ein Reinigungsaufwand für das Instrumentarium deutlich verringert werden. Insbesondere können die Führungsschlitze parallel oder im Wesentlichen parallel zueinander angeordnet oder ausgebildet sein.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische Ansicht eines ersten Ausführungsbeispiels eines erfindungsgemäßen medizinischen Instrumentariums;
- Figur 2:: eine weitere perspektivische Gesamtansicht des medizinischen Instrumentariums aus Figur 1;
- Figur 3:: eine Explosionsdarstellung der Anordnung aus Figur 2;

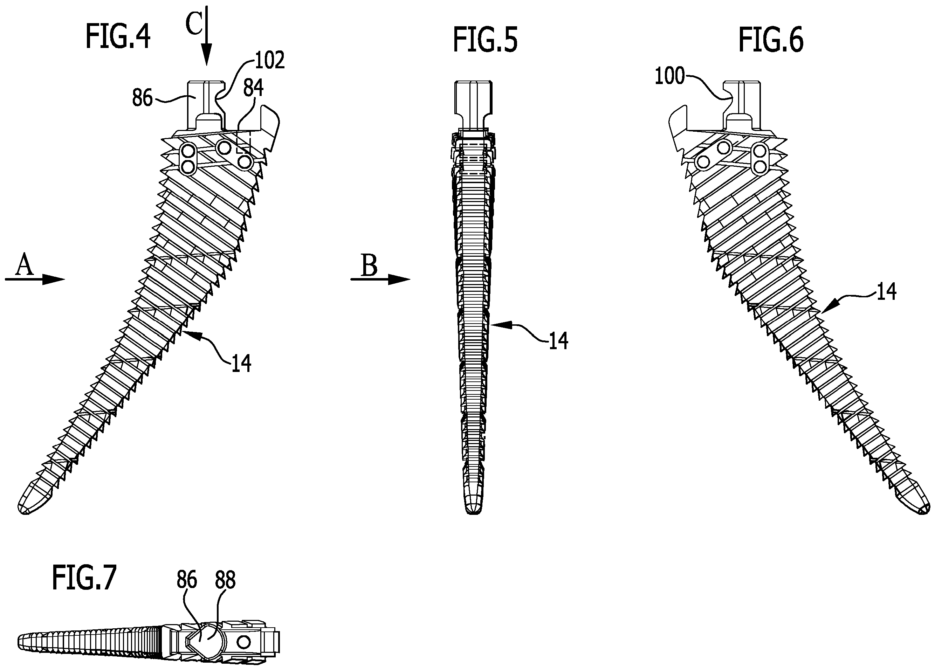

- Figur 4:: eine Seitenansicht des Raspelkörpers des Instrumentariums aus den Figuren 1 bis 3;
- Figur 5:: eine Ansicht des Raspelkörpers aus Figur 4 in Richtung des Pfeils A;
- Figur 6:: eine Seitenansicht des Raspelkörpers aus Figur 5 in Richtung des Pfeils B;
- Figur 7:: eine Ansicht des Raspelkörpers aus Figur 4 in Richtung des Pfeils C;
- Figur 8:: eine Seitenansicht des Kopplungskörpers des Instrumentariums aus den Figuren 1 bis 3;
- Figur 9:: eine teilweise durchbrochene Seitenansicht des Kopplungskörpers aus Figur 8;
- Figur 10:: eine Ansicht des Kopplungskörpers aus Figur 8 in Richtung des Pfeils E;
- Figur 11:: eine Seitenansicht des Raspelkörpers ähnlich Figur 4 mit gekoppeltem Kopplungskörper in einer Längsschnittansicht;
- Figur 12:: eine Seitenansicht des Schablonenkörpers aus den Figuren 1 bis 3;
- Figur 13:: eine Draufsicht auf den Schablonenkörper aus Figur 12 in Richtung des Pfeils F;
- Figur 14:: eine Seitenansicht eines zweiten Ausführungsbeispiels eines Schablonenkörpers;
- Figur 15:: eine Draufsicht auf den Schablonenkörper aus Figur 14 in Richtung des Pfeils G;
- Figur 16:: eine schematische perspektivische und teilweise durchbrochene Gesamtansicht eines zweiten Ausführungsbeispiels einer Sägeschablone;
- Figur 17:: eine Schnittansicht längs Linie 17-17 in Figur 16; und
- Figur 18:: eine Explosionsdarstellung der Sägeschablone aus Figur 16.

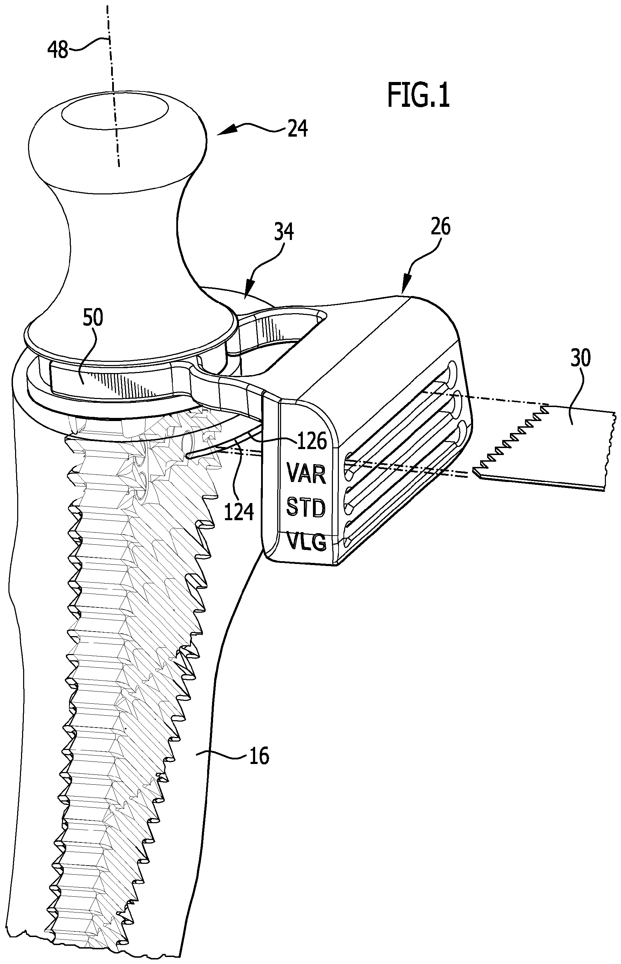

In den Figuren 1 bis 3 ist ein erstes Ausführungsbeispiel eines insgesamt mit dem Bezugszeichen 10 bezeichneten medizinischen Instrumentariums zum Implantieren einer Hüftgelenkendoprothese schematisch dargestellt. Das Instrumentarium umfasst mindestens eine Sägeschablone 12 und optional einen Raspelkörper 14 einer Prothesenraspel zum Präparieren einer Kavität eines schematisch in Figur 1 dargestellten Femurknochens, in welche Kavität ein zu implantierender Prothesenschaft der Hüftgelenkendoprothese eingesetzt wird.

Die Sägeschablone 12 umfasst ein erstes Kopplungselement 18 zum temporären Koppeln der Sägeschablone 12 mit einem zweiten Kopplungselement 20 des Raspelkörpers 14 in einer Kopplungsstellung. Die Kopplungsstellung ist beispielsweise in den Figuren 1, 2 und 11 schematisch dargestellt.

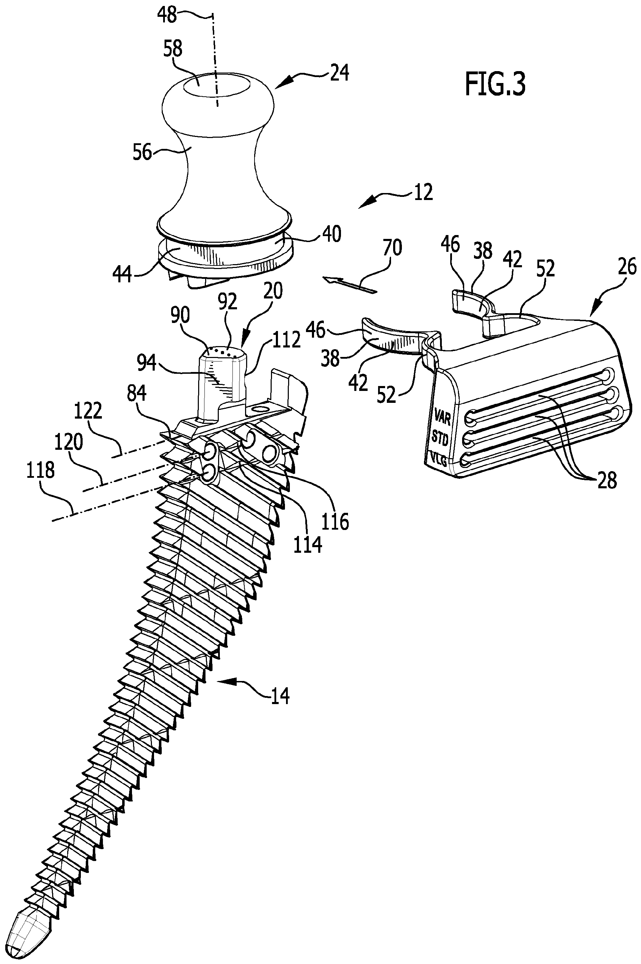

Die ersten und zweiten Kopplungselemente 18 und 20 bilden zusammen eine Kopplungseinrichtung 22. In der Kopplungsstellung stehen die ersten und zweiten Kopplungselemente 18 und 20 kraft- und/oder formschlüssig in Eingriff. In einer Reinigungsstellung, wie sie schematisch in Figur 3 dargestellt ist, sind die ersten und zweiten Kopplungselemente 18 und 20 vollständig voneinander getrennt. Mithin sind auch die Sägeschablone 12 und der Raspelkörper 14 in der Reinigungsstellung vollständig voneinander getrennt.

Die Sägeschablone 12 umfasst einen Kopplungskörper 24 und einen Schablonenkörper 26.

Das erste Kopplungselement 18 ist am Kopplungskörper 24 angeordnet oder ausgebildet.

Am Schablonenkörper 26 ist mindestens ein Führungsschlitz 28 für ein Sägeblatt 30 einer in den Zeichnungen nicht näher dargestellten chirurgischen Säge angeordnet oder ausgebildet. Es können auch zwei oder mehr Führungsschlitze 28 am Schablonenkörper 26 vorgesehen sein. Bei dem in den Figuren 1 bis 3 schematisch dargestellten Ausführungsbeispiel sind insgesamt drei Führungsschlitze 28 angeordnet beziehungsweise ausgebildet.

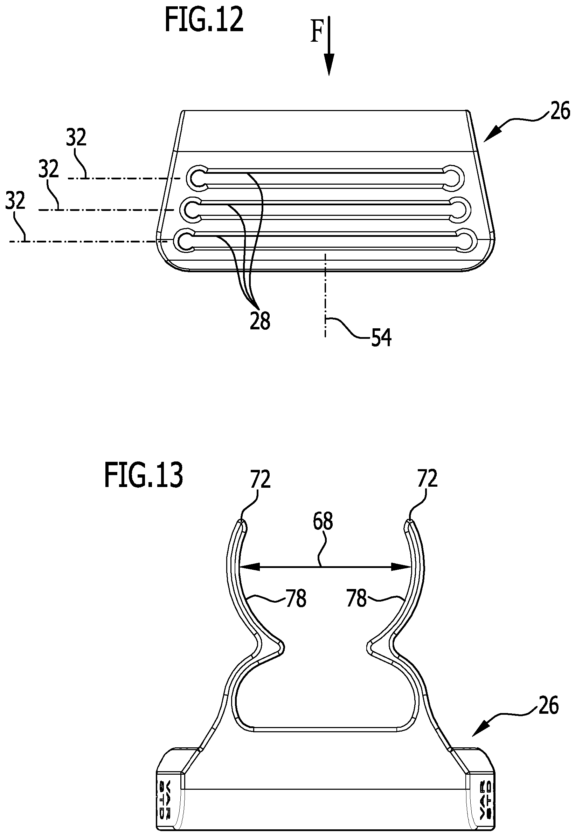

Jeder Führungsschlitz 28 definiert eine Führungsebene 32. Diese Führungsebenen 32 verlaufen bei dem in den Figuren 1 bis 3 und 12 schematisch dargestellten Ausführungsbeispiel des Schablonenkörpers 26 insbesondere parallel zueinander.

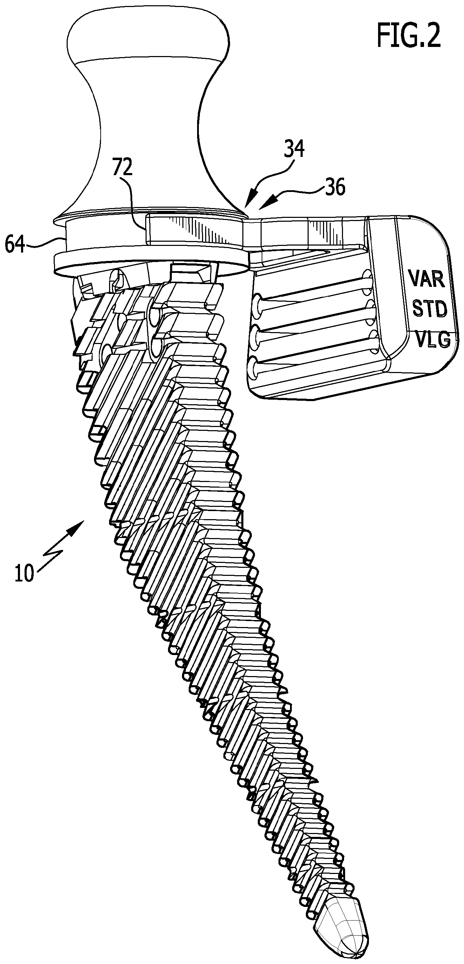

Das Instrumentarium 10 umfasst ferner eine Kupplungseinrichtung 34 zum temporären Kuppeln des Kopplungskörpers 24 mit dem Schablonenkörper 26 in einer Kupplungsstellung. Die Kupplungsstellung ist beispielhaft in den Figuren 1 und 2 schematisch dargestellt.

Die Kupplungseinrichtung 34 ist in Form einer Rast- oder Schnappverbindungseinrichtung 36 ausgebildet und umfasst in der Kupplungsstellung miteinander in Eingriff stehende erste und zweite Rast- oder Schnappelemente 38 und 40.

Die Kupplungseinrichtung 34 umfasst mithin erste und zweite Kupplungselemente 42 und 44, die die Rast- oder Schnappelemente 38 und 40 bilden.

Die ersten und zweiten Kupplungselemente 42 und 44 sind einerseits am Schablonenkörper 26, nämlich die ersten Kupplungselemente 42, und andererseits am Kopplungskörper 24, nämlich das zweite Kupplungselement 44, angeordnet oder ausgebildet. Die ersten und zweiten Kupplungselemente 42 und 44 stehen in der Kupplungsstellung kraft- und/oder formschlüssig miteinander in Eingriff.

Die ersten Kupplungselemente sind in Form von Kupplungsvorsprüngen 46 ausgebildet, und zwar als auf eine von der Kopplungseinrichtung 22 definierte Kopplungslängsachse hin weisend konkav gekrümmte Haltebügel 50.

Die Kupplungsvorsprünge 46 sind federnd, beispielsweise über einen blattfederartig ausgebildeten Verbindungsabschnitt 52, mit dem Schablonenkörper 26 verbunden.

Insgesamt ist der Schablonenkörper 26 einstückig ausgebildet. Er ist zudem spiegelsymmetrisch zu einer Symmetrieebene 54 ausgebildet, welche senkrecht zu den Führungsebenen 32 verläuft.

Der Kopplungskörper 24 ist insgesamt im Wesentlichen rotationssymmetrisch bezogen auf die Kopplungslängsachse 48 ausgebildet und definiert einen Griffbereich 56, welcher sich bis zu einem proximalen Ende 58 des Kopplungskörpers 24 hin erstreckt.

Benachbart einem distalen Ende 60 ist am Kopplungskörper 24 eine Kupplungsausnehmung 62 ausgebildet, welche zu den Kupplungsvorsprüngen 56 korrespondiert.

Die Kupplungsausnehmung 62 ist in Form einer Ringnut 64 ausgebildet, welche von der Kopplungslängsachse 48 weg weisend geöffnet ist. Ein Außendurchmesser 66 des Kopplungskörpers 24 im Bereich der Ringnut 64 in einer Ebene senkrecht zur Kopplungslängsachse 48 ist etwas größer als ein von den beiden Haltebügeln 50 definierter Innendurchmesser 68 in einer Stellung, in der der Kopplungskörper 24 und der Schablonenkörper 26 voneinander getrennt sind.

Zum Kuppeln des Kopplungskörpers 24 und des Schablonenkörpers 26 wird der Schablonenkörper 26 mit den Haltebügeln 50 voran in Richtung des Pfeils 70 mit freien Enden 72 der Haltebügel 50 parallel zu einer von der Ringnut 64 definierten Einführebene 74 an die Ringnut 64 herangeführt. Dabei gleiten die Enden 72 an der vom Kopplungskörper 24 weg weisenden zylindrischen Nutbodenfläche 76 auf, so dass die Haltebügel 50 voneinander weg gespreizt werden. Dabei verformen sich die Verbindungsabschnitte 52 etwas voneinander weg, bis die Nutbodenfläche 76 sich an in Richtung auf die Kopplungslängsachse 48 hin weisende Innenflächen 78 der Haltebügel 50 anlegen kann. Die Verbindungsabschnitte 52 halten die Haltebügel 50 etwas unter Vorspannung in der Ringnut 64.

Die beschriebene Ausgestaltung der Kupplungseinrichtung 34 ermöglicht eine Verdrehung des Kopplungskörpers 24 und des Schablonenköpers 26 in der Kupplungsstellung relativ zueinander um eine durch die Kopplungslängsachse 48 definierte Drehachse 80.

Bei dem in den Figuren dargestellten Ausführungsbeispiel des Instrumentariums 10 verlaufen die Führungsebenen 32, quer, insbesondere senkrecht, zur Drehachse 80.

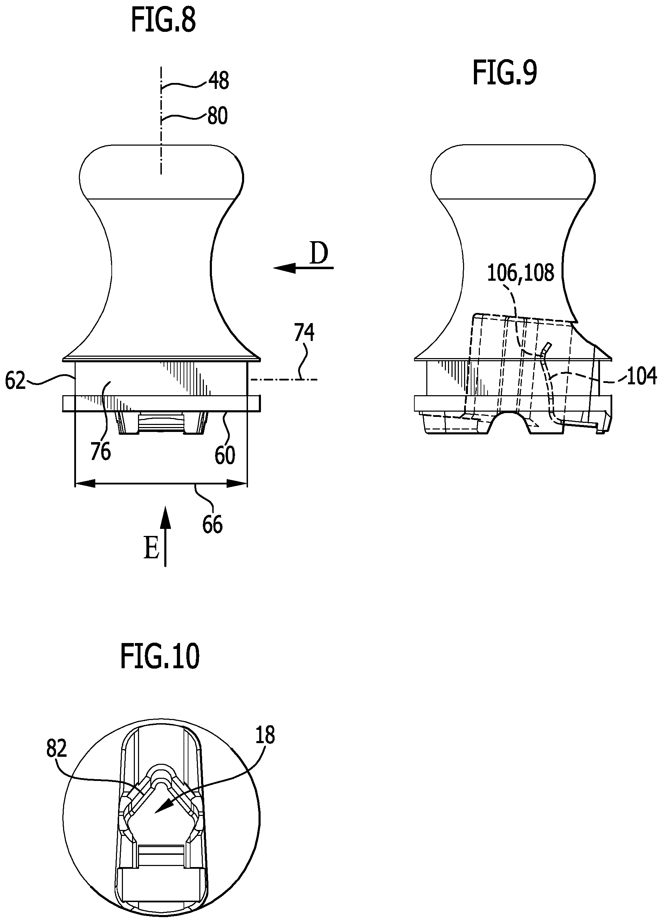

Das erste Kopplungselement 18 ist in Form einer Kopplungselementaufnahme 82 ausgebildet. Ein freier Querschnitt der Kopplungselementaufnahme 82 in einer Schnittebene quer zur Kopplungslängsachse 48, wie schematisch in Figur 10 dargestellt, ist im Wesentlichen rautenförmig.

Das zweite Kopplungselement 20 ist am Raspelkörper 14 von einer proximalen Endfläche 84 desselben in proximaler Richtung weisend abstehend ausgebildet und definiert einen Kopplungsvorsprung 86. Eine Querschnittfläche 88 des Kopplungsvorsprungs 46 in einer Ebene quer zur Kopplungslängsachse 48 setzt sich aus einem gleichschenkligen Dreieck 90 und einem Halbkreis 92 zusammen, wobei eine Basis des Dreiecks 90 einen Halbmesser des Halbkreises 92 definiert.

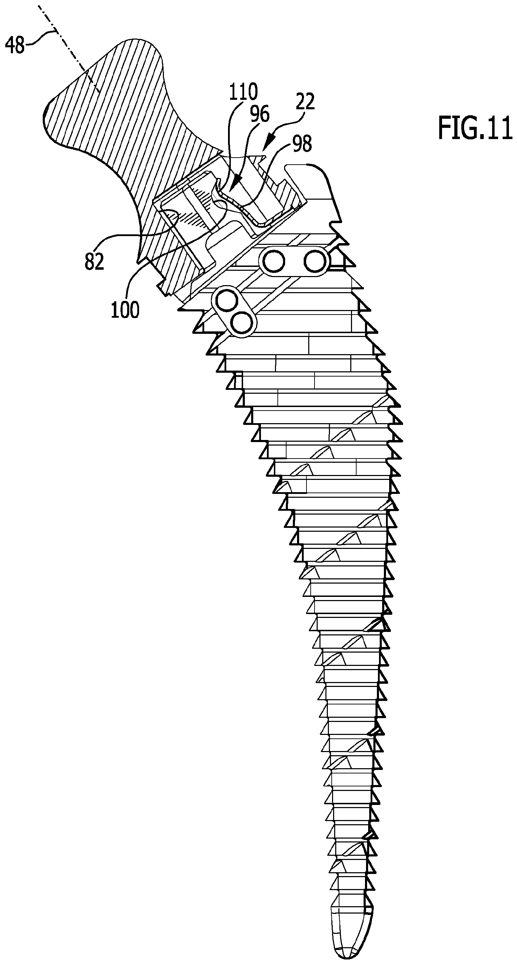

Der Kopplungsvorsprung 86 ist somit durch einen rotationsunsymmetrischen Kopplungszapfen 94 gebildet, welcher in der Kopplungsstellung vollständig in die Kopplungselementaufnahme 82 am Kopplungskörper 24 eintauchen kann, wie dies beispielhaft in Figur 11 dargestellt ist. Durch die korrespondierende Ausbildung der Kopplungselementaufnahme 82 und des Kopplungsvorsprungs 86 sind die ersten und zweiten Kopplungselemente 18 und 20 in der Kopplungsstellung drehfest miteinander in Eingriff stehend.

Um die Sägeschablone 12 am Raspelkörper 14 in der Kopplungsstellung zu sichern, ist eine Sicherungseinrichtung 96 vorgesehen, welche ein erstes Sicherungselement 98 umfasst, welches dem ersten Kopplungselement 18 zugeordnet ist.

Das erste Sicherungselement 98 steht in einer Sicherungsstellung mit einem zweiten Sicherungselement 100 kraft- und/oder formschlüssig in Eingriff. Das zweite Sicherungselement 100 ist am Raspelkörper 14 angeordnet beziehungsweise ausgebildet.

Das Sicherungselement 98 ist mithin am ersten Kopplungselement 80 angeordnet oder ausgebildet. Das zweite Sicherungselement 100 ist am zweiten Kopplungselement 20 angeordnet oder ausgebildet. Das zweite Sicherungselement 100 ist in Form einer Rastausnehmung 102 am Kopplungszapfen 94 ausgebildet und in einer Richtung quer zur Kopplungslängsachse 48 weisend geöffnet.

Das erste Sicherungselement 98 ist in Form eines Blattfederbügels 104 mit einer in Richtung auf die Rastausnehmung 102 hin weisenden Rastnase 106 ausgebildet, welche in der Sicherungsstellung in die Rastausnehmung 102 eintaucht. Dies ist jedoch nur dann möglich, wenn die Kopplungselemente 18 und 20 die Kopplungsstellung einnehmen.

Die Rastnase 106 bildet einen Rastvorsprung 108, welcher ein erstes Rastglied 110 bildet. Ein mit diesem zusammenwirkendes zweites Rastglied 112 wird durch die Rastausnehmung 102 gebildet.

Die beschriebene Ausgestaltung der Rastglieder 110 und 112 ermöglicht es insbesondere, dass der Rastvorsprung 108 beim in Eingriff Bringen des ersten Kopplungselements 18 und des zweiten Kopplungselements 20 von einer Grundstellung, wie sie schematisch in Figur 9 dargestellt ist, in eine von der Kopplungslängsachse 48 weg nach außen ausgelenkte Stellung bewegt wird und erst dann, wenn die Kopplungseinrichtung 22 die Kopplungsstellung einnimmt, sich von dieser ausgelenkten Stellung zurück bewegen und in die Rastausnehmung 102 eingreifen kann, so dass die Sicherungseinrichtung 96 die Sicherungsstellung einnimmt, in der die Sicherungselemente 98 und 100 miteinander kraft- und/oder formschlüssig in Eingriff stehen.

Die drei Führungsschlitze 28 am Schablonenkörper 26 sind durch Buchstabenkombinationen gekennzeichnet. Die Buchstabenkombinationen "VAR", "STD" und "VLG" bilden Abkürzungen der Begriffe "Varus", "Standard" und "Valgus". Die drei Führungsschlitze 28 korrespondieren zu nutförmigen Markierungen 114, 116 sowie zur Endfläche 84 am Raspelkörper 14, die jeweils parallel zueinander verlaufende Einschlagebenen 118, 120 und 122, definieren.

Wird der Raspelkörper 14 in die Kavität am Femurknochen 16 eingeschlagen, so definieren die Markierungen 114, 116 sowie die Endfläche 84 Einschlagtiefen für das vom Operateur für den Patienten ausgewählte Implantat, nämlich den jeweiligen Prothesenschaft. Für eine Korrektur einer Valgus-Stellung wird ein Prothesenschaft verwendet, der zur Markierung 114 korrespondiert. Für einen Standardprothesenschaft wird die Markierung 116 genutzt. Zur Korrektur einer Varus-Stellung wird die Endfläche 84 genutzt. Auf diese Weise lässt sich die Femurkavität mit einem einzigen Raspelkörper 14 für drei unterschiedliche Prothesenformen vorbereiten.

Ist der Raspelkörper 14 vom Operateur mit Hilfe eines nicht dargestellten, mit dem zweiten Kopplungselement 20 gekoppelten Handgriff in den Femurknochen 16 durch Einschlagen eingebracht, kann dieser Handgriff entfernt und das zweite Kopplungselement 20 mit dem Kopplungskörper 24 in der beschriebenen Weise drehfest und axial bezogen auf die Kopplungsachse 48 gekoppelt werden.

Falls der Schablonenkörper 26 dabei noch nicht mit dem Kopplungskörper 24 gekoppelt ist, kann er nun in der oben beschriebenen Weise mit dem Kopplungskörper 24 in Eingriff gebracht werden. Der Operateur kann dann mit einer Säge den Femurknochen 16 so weit resezieren, dass eine optimal auf den einzusetzenden Prothesenschaft abgestimmte Femurendfläche 126 ausgebildet wird. Hierfür führt der Operateur das Sägeblatt 30 durch den entsprechenden Führungsschlitz 28 und setzt den Sägeschnitt 124 am Femurknochen 16. In Figur 1 ist dies beispielhaft dargestellt für einen eine Varus-Stellung korrigierenden Prothesenschaft. Das Sägeblatt 30 wird durch den mit "VAR" bezeichneten Führungsschlitz 28 geführt und ein entsprechender Sägeschnitt 120 am Femurknochen 16 ausgebildet.

Durch die Drehbarkeit des Schablonenkörpers 26 relativ zum Kopplungskörper 24 können mehrere Sägeschnitte 124 aus verschiedenen Richtungen angesetzt werden.

Ist der Femurknochen 16 in der beschriebenen Weise mit Sägeschnitten 124 versehen, kann die Sägeschablone 12 vom Raspelkörper 14 wieder abgezogen werden. Der Raspelkörper 14 wird dann aus dem Femurknochen 16 heraus gezogen. Der Femurknochen 16 kann dann mit der Knochensäge weiter bearbeitet werden. Die Sägeschnitte 124 dienen dabei als Führung für das Sägeblatt 30, mit dem dann der Femurknochen 16 abschließend zur Ausbildung der Femurendfläche 126 bearbeitet wird.

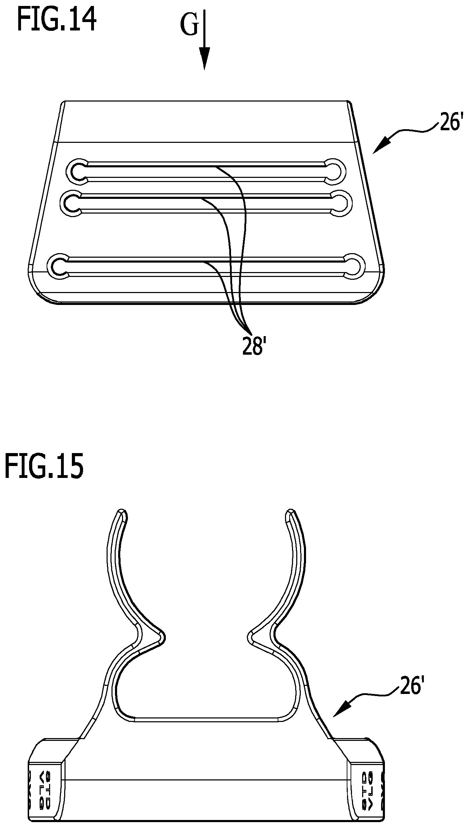

In den Figuren 14 und 15 ist ein zweites Ausführungsbeispiel eines Schablonenkörpers 26' schematisch dargestellt. Er stimmt in seinem Aufbau im Wesentlichen mit dem Schablonenkörper 26 überein. Insbesondere die ersten Kupplungselemente 42 zum Kuppeln des Schablonenkörpers 26' mit dem Kopplungskörper 24 sind identisch ausgebildet.

Der Kopplungskörper 26' unterscheidet sich vom Kopplungskörper 26 durch die Abstände der Führungsschlitze 28'. Dieser Schablonenkörper 26' kann insbesondere dann zum Einsatz kommen, wenn ein anderer Raspelkörper 14 zum Präparieren der Femurkavität genutzt wird, mit dem beispielsweise die Femurkavität für einen "Standard"-Prothesenschaft vorbereitet werden soll oder für einen Prothesenschaft zur Korrektur einer "Valgus"-Stellung oder für einen Prothesenschaft zur Korrektur einer displastischen Fehlstellung.

Mithin kann also der Schablonenkörper 26' mit dem Kopplungskörper 24 in analoger Weise wie oben beschrieben gekoppelt werden zum Präparieren des Femurknochens in Verbindung mit einem sich vom Raspelkörper 14 unterscheidenden, in den Figuren nicht dargestellten weiteren Raspelkörper.

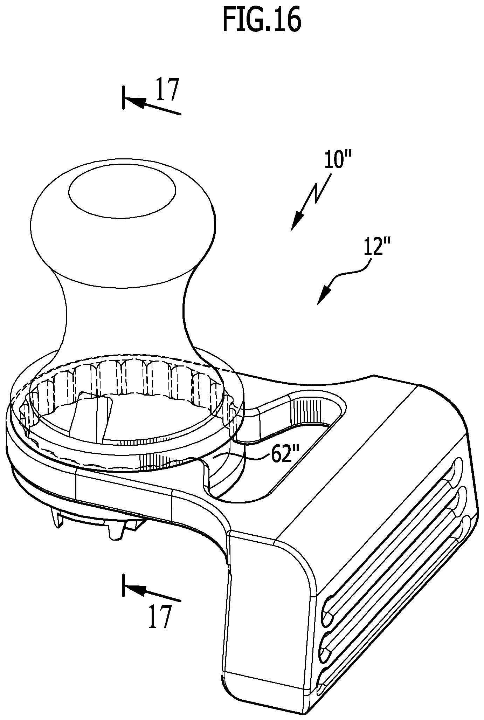

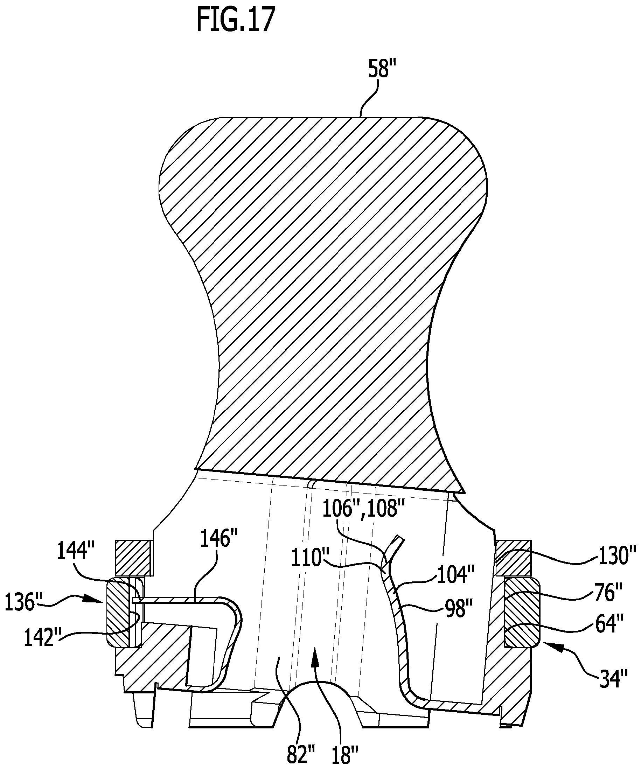

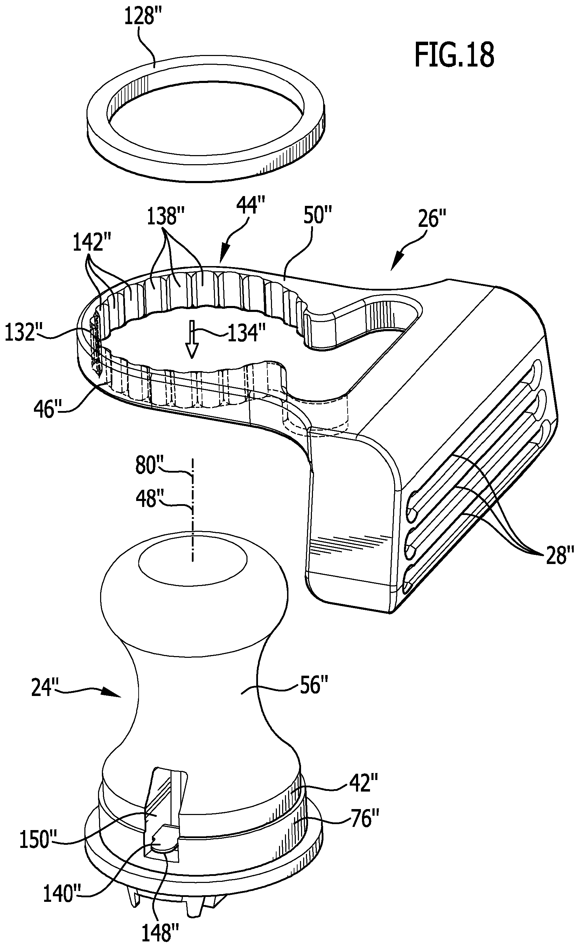

In den Figuren 16 bis 18 ist ein zweites Ausführungsbeispiel eines insgesamt mit den Bezugszeichen 10" bezeichneten Instrumentariums schematisch dargestellt. Es umfasst einen Kopplungskörper 24" sowie einen Schablonenkörper 26".

Der Kopplungskörper 24" stimmt in seinem Aufbau im Wesentlichen mit dem Kopplungskörper 24 überein. Allerdings ist die Ringnut 64" in Richtung auf das Ende 58" hin weisend seitlich offen, kann jedoch durch einen Haltering 128" seitlich begrenzt und dadurch seitlich geschlossen werden.

Der Haltering 128" und der Kopplungskörper 24" können insbesondere kraft- und/oder form- und/oder stoffschlüssig miteinander verbunden sein. Insbesondere kann am Kopplungskörper benachbart zur Ringnut 64" ein ringförmiger Rücksprung 130" ausgebildet sein, in den der Haltering 128 eingreift, wenn er die Ringnut 64" seitlich verschließt.

Das erste Kopplungselement 18" am Kopplungskörper 24" ist im Wesentlichen identisch ausgebildet mit dem ersten Kopplungselement 18. Ferner ist dem ersten Kopplungselement 18" ein erstes Sicherungselement 98" zugeordnet, das identisch ausgebildet und angeordnet ist wie das erste Sicherungselement 98 am ersten Kopplungselement 18.

Der Kopplungskörper 24" kann so in identischer Weise wie oben beschrieben mit dem Raspelkörper 14 gekoppelt werden.

Der Schablonenkörper 26" unterscheidet sich vom Schablonenkörper 26 im Wesentlichen durch die Ausgestaltung des zweiten Kupplungselements 44". Dieses ist in Form eines nahezu in sich geschlossenen, sich über mindestens 180° in Umfangsrichtung bezogen auf die durch die Kopplungslängsachse 48" definierte Drehachse 80" erstreckenden Kupplungsrings 132" ausgebildet.

Zum Kuppeln des Schablonenkörpers 26" mit dem Kopplungskörper 24" wird der Kupplungsring 132" in Richtung des Pfeils 134" parallel zur Kopplungslängsachse 48" über den Griffbereich 56" geschoben, bis der Kupplungsring 132" die Nutbodenfläche 76" umgebend angeordnet ist.

Zum Sichern des Kupplungsrings 132" in der Ringnut 64" dient wie beschrieben der Haltering 128", der ebenfalls parallel zum Pfeil 134 über den Griffbereich 56" vom Ende 58" her kommend geschoben und an den Rücksprung 130" herangeführt und dort befestigt wird. Insbesondere kann der Haltering 128" mit dem Rücksprung 130" verschweißt, verlötet oder verklebt werden.

Das Instrumentarium 10" umfasst ferner eine Winkeleinstelleinrichtung 136" zum Einstellen einer Winkelstellung des Kopplungskörpers 24" und des Schablonenkörpers 26" bezogen auf die Drehachse 80" relativ zueinander. Dies wird insbesondere erreicht, indem die ersten und zweiten Kupplungselemente 42" und 44" relativ zueinander in diskreten Winkelstellungen bezogen auf die Drehachse 80" ausrichtbar sind.

Die Winkeleinstelleinrichtung 136" umfasst eine Mehrzahl erster Winkeleinstellglieder 138" und mindestens ein zweites Winkeleinstellglied 140". Das zweite Winkeleinstellglied 140" ist in unterschiedlichen Winkelstellung mit unterschiedlichen ersten Winkeleinstellgliedern 138" in Eingriff stehend.

Die Mehrzahl erster Winkeleinstellglieder 138" ist in Form von Winkelgliedausnehmungen 142" ausgebildet. Das zweite Winkeleinstellglied 140" ist in Form eines Winkelgliedvorsprungs 144" ausgebildet.

Die Winkelgliedausnehmungen 142" sind am zweiten Kupplungselement 44" angeordnet oder ausgebildet und in Richtung auf die Drehachse 80" hin weisend geöffnet. Sie bilden eine Art in Richtung auf die Drehachse 80" hin weisende Verzahnung am Kupplungsring 132".

Der Winkelgliedvorsprung 144" ist federnd ausgebildet beziehungsweise gelagert, und zwar bildet er ein freies Ende eines Federbügels 146" am Kopplungskörper 24". Das freie Ende 148" ragt von der Drehachse 80" weg weisend durch eine Durchbrechung 150" am Kopplungskörper 24" nach außen vor, so dass der Winkelgliedvorsprung 124" in eine der Winkelgliedausnehmungen 142" eintauchen kann.

Beim Ändern der Winkelstellung zwischen dem Kopplungskörper 24" und dem Schablonenkörper 26" durch Drehen derselben um die Drehachse 80" wird der Federbügel 146" entgegen einer von ihm ausgeübten vorspannenden Kraft aus einer der Winkelgliedausnehmungen 142" hinaus bewegt und bei entsprechender Verdrehung durch die im Federbügel 146" gespeicherte vorspannende Kraft in eine benachbarte Winkelgliedausnehmung 142" zurück bewegt. So lassen sich durch entsprechende Größe beziehungsweise Breite der Winkelgliedausnehmungen 142" und des zugehörigen Winkelgliedvorsprungs 144" unterschiedliche Anzahlen von Winkelstellungen definieren.

Beim Verdrehen des Schablonenkörpers 26" um die Drehachse 80" relativ zum Kopplungskörper 24" kann ein Operateur diese unterschiedlichen Rastungen zudem erspüren.

Die oben beschriebenen Instrumentarien 10 und 10" können insbesondere vollständig aus heißdampfsterilisierbaren metallischen Werkstoffen ausgebildet werden.

Wahlweise kann an den Haltebügeln 50 des Schablonenkörpers 26 auch eine Verzahnung ähnlich der Winkelgliedausnehmungen 142" vorgesehen werden, so dass ein derart modifizierter Schablonenkörper 26 optional auch mit dem Kopplungskörper 24" zusammen genutzt werden kann zum Einstellen definierter Winkelstellungen des so modifizierten Schablonenkörpers und des Kopplungskörpers 24" bezogen auf die Drehachse relativ zueinander.

### Bezugszeichenliste

- 10, 10": Instrumentarium
- 12, 12": Sägeschablone
- 14: Raspelkörper
- 16: Femurknochen
- 18, 18": erstes Kopplungselement
- 20: zweites Kopplungselement
- 22: Kopplungseinrichtung
- 24, 24": Kopplungskörper
- 26, 26', 26": Schablonenkörper
- 28, 28', 28": Führungsschlitz
- 30: Sägeblatt
- 32: Führungsebene
- 34, 34": Kupplungseinrichtung
- 36: Rast- oder Schnappverbindungseinrichtung
- 38: erstes Rast- oder Schnappelement
- 40: zweites Rast- oder Schnappelement
- 42, 42": erstes Kupplungselement
- 44, 44": zweites Kupplungselement
- 46, 46": Kupplungsvorsprung
- 48, 48": Kopplungslängsachse
- 50, 50": Haltebügel
- 52: Verbindungsabschnitt
- 54: Symmetrieebene
- 56, 56": Griffbereich
- 58, 58": Ende
- 60: Ende
- 62, 62": Kopplungsausnehmung
- 64, 64": Ringnut
- 66: Außendurchmesser
- 68: Innendurchmesser
- 70: Pfeil
- 72: Ende
- 74: Einführebene
- 76, 76": Nutbodenfläche
- 78: Innenfläche
- 80, 80": Drehachse
- 82, 82": Kopplungselementaufnahme
- 84: Endfläche
- 86: Kopplungsvorsprung
- 88: Querschnittsfläche
- 90: Dreieck
- 92: Halbkreis
- 94: Kopplungszapfen
- 96: Sicherungseinrichtung
- 98, 98": erstes Sicherungselement
- 100: zweites Sicherungselement
- 102: Rastausnehmung
- 104, 104": Blattfederbügel
- 106, 106": Rastnase
- 108, 108": Rastvorsprung
- 110, 110": erstes Rastglied
- 112: zweites Rastglied
- 114: Markierung
- 116: Markierung
- 118: Einschlagebene
- 120: Einschlagebene
- 122: Einschlagebene
- 124: Sägeschnitt
- 126: Femurendfläche
- 128": Haltering
- 130": Rücksprung
- 132": Kupplungsring
- 134": Pfeil
- 136": Winkeleinstelleinrichtung
- 138": erstes Winkeleinstellglied
- 140": zweites Winkeleinstellglied
- 142": Winkelgliedausnehmung
- 144": Winkelgliedvorsprung
- 146": Federbügel
- 148": Ende
- 150": Durchbrechung

## Patentansprüche

1. Medizinisches Instrumentarium (10; 10") zum Implantieren einer Hüftgelenkendoprothese, welches Instrumentarium (10; 10") mindestens eine Sägeschablone (12; 12") mit mindestens einem Führungsschlitz (28; 28'; 28") für ein Sägeblatt (30) umfasst, wobei die Sägeschablone mindestens ein erstes Kopplungselement (18; 18") zum temporären Koppeln der Sägeschablone (12; 12") mit einem zweiten Kopplungselement (20) eines Raspelkörpers (14) einer Prothesenraspel in einer Kopplungsstellung umfasst, wobei dem ersten Kopplungselement (18; 18") ein erstes Sicherungselement (98; 98") einer Sicherungseinrichtung (96) zugeordnet ist, welches in der Kopplungsstellung mit einem am Raspelkörper (14) angeordneten zweiten Sicherungselement (100) in einer Sicherungsstellung kraft- und/oder formschlüssig in Eingriff steht, **dadurch gekennzeichnet, dass** das erste und das zweite Sicherungselement (98, 100; 98", 100) in Form von zusammenwirkenden Rastgliedern (110, 112) ausgebildet sind.

2. Medizinisches Instrumentarium nach Anspruch 1, **dadurch gekennzeichnet, dass** die Sägeschablone (12; 12") einen Kopplungskörper (24; 24") und einen Schablonenkörper (26; 26'; 26") umfasst und dass das mindestens eine erste Kopplungselement (18; 18") am Kopplungskörper (24; 24") angeordnet oder ausgebildet ist und dass der mindestens eine Führungsschlitz (28; 28'; 28") am Schablonenkörper (26; 26'; 26") angeordnet oder ausgebildet ist.

3. Medizinisches Instrumentarium nach Anspruch 2, **gekennzeichnet durch** eine Kupplungseinrichtung (34; 34") zum temporären Kuppeln des Kopplungskörpers (24; 24") und des Schablonenkörpers (26; 26'; 26") in einer Kupplungsstellung.

4. Medizinisches Instrumentarium nach Anspruch 3, **dadurch gekennzeichnet, dass**
a) die Kupplungseinrichtung (34) in Form einer Rast- oder Schnappverbindungseinrichtung (36) ausgebildet ist und in der Kupplungsstellung miteinander in Eingriff stehende Rast- oder Schnappelemente (38, 40) umfasst
und/oder
b) die Kupplungseinrichtung (34; 34") erste und zweite Kupplungselemente (42, 44; 42", 44") umfasst, dass die ersten und zweiten Kupplungselemente (42, 44; 42", 44") einerseits am Kopplungskörper (24; 24") und andererseits am Schablonenkörper (26; 26'; 26") angeordnet oder ausgebildet sind und dass die ersten und zweiten Kupplungselemente (42, 44; 42", 44") in der Kupplungsstellung kraft- und/oder formschlüssig in Eingriff stehen,
wobei insbesondere die ersten und zweiten Kupplungselemente (42, 44) die Rast- oder Schnappelemente (38, 40) bilden,
und/oder
c) der Kopplungskörper (24; 24") und der Schablonenkörper (26; 26'; 26") in der Kupplungsstellung relativ zueinander um eine durch die Kupplungseinrichtung (34; 34") definierte Drehachse (80; 80") verdrehbar aneinander gelagert sind.

5. Medizinisches Instrumentarium nach Anspruch 3 oder 4, **dadurch gekennzeichnet, dass** die Kupplungseinrichtung (34; 34") erste und zweite Kupplungselemente (42, 44; 42", 44") umfasst, dass die ersten und zweiten Kupplungselemente (42, 44; 42", 44") einerseits am Kopplungskörper (24; 24") und andererseits am Schablonenkörper (26; 26'; 26") angeordnet oder ausgebildet sind und dass die ersten und zweiten Kupplungselemente (42, 44; 42", 44") in der Kupplungsstellung kraft- und/ oder formschlüssig in Eingriff stehen, dass die ersten und zweiten Kupplungselemente (42, 44; 42", 44") in Form mindestens eines Kupplungsvorsprungs (46; 46") und in Form einer korrespondierenden Kupplungsausnehmung (62; 62") ausgebildet sind.

6. Medizinisches Instrumentarium nach Anspruch 5, **dadurch gekennzeichnet, dass**
a) der mindestens eine Kupplungsvorsprung (46) federnd am Schablonenkörper (26) angeordnet oder ausgebildet ist
und/oder
b) der mindestens eine Kupplungsvorsprung (46) in Form eines auf die Drehachse hin weisend konkav gekrümmten Haltebügels (50) ausgebildet ist
und/oder
c) die mindestens eine Kupplungsausnehmung (62; 62") in Form einer von der Drehachse (80; 80") weg weisend geöffneten Ringnut (64; 64") ausgebildet ist und dass der mindestens eine Kupplungsvorsprung in der Kupplungsstellung in Umfangsrichtung bezogen auf die Drehachse (80; 80") mindestens abschnittsweise in die Kupplungsausnehmung (62; 62") eingreift.

7. Medizinisches Instrumentarium nach Anspruch 5 oder 6, **dadurch gekennzeichnet, dass** der Kopplungskörper (24; 24") und der Schablonenkörper (26; 26'; 26") in der Kupplungsstellung relativ zueinander um eine durch die Kupplungseinrichtung (34; 34") definierte Drehachse (80; 80") verdrehbar aneinander gelagert sind und dass der mindestens eine Kupplungsvorsprung (46") in Form eines in sich geschlossenen oder sich über mindestens 180° in Umfangsrichtung bezogen auf die Drehachse (80") erstreckenden Kupplungsrings (132") ausgebildet ist.

8. Medizinisches Instrumentarium nach einem der Ansprüche 5 bis 7, **dadurch gekennzeichnet, dass** die mindestens eine Kupplungsausnehmung (62; 62") in Form einer von der Drehachse (80; 80") weg weisend geöffneten Ringnut (64; 64") ausgebildet ist und dass der mindestens eine Kupplungsvorsprung in der Kupplungsstellung in Umfangsrichtung bezogen auf die Drehachse (80; 80") mindestens abschnittsweise in die Kupplungsausnehmung (62; 62") eingreift und dass die Ringnut (64") seitlich von einem Haltering (128") begrenzt ist,
wobei insbesondere der Haltering (128") und der Kopplungskörper (24") kraft- und/oder form- und/oder stoffschlüssig miteinander verbunden sind.

9. Medizinisches Instrumentarium nach einem der Ansprüche 5 bis 8,
**dadurch gekennzeichnet, dass** die mindestens eine Kupplungsausnehmung (62; 62") in Form einer von der Drehachse (80; 80") weg weisend geöffneten Ringnut (64; 64") ausgebildet ist und dass der mindestens eine Kupplungsvorsprung in der Kupplungsstellung in Umfangsrichtung bezogen auf die Drehachse (80; 80") mindestens abschnittsweise in die Kupplungsausnehmung (62; 62") eingreift und dass die ersten und zweiten Kupplungselemente (42", 44") relativ zueinander in diskreten Winkelstellungen bezogen auf die Drehachse (80") ausrichtbar sind.

10. Medizinisches Instrumentarium nach einem der Ansprüche 5 bis 9, **dadurch gekennzeichnet, dass** die mindestens eine Kupplungsausnehmung (62; 62") in Form einer von der Drehachse (80; 80") weg weisend geöffneten Ringnut (64; 64") ausgebildet ist und dass der mindestens eine Kupplungsvorsprung in der Kupplungsstellung in Umfangsrichtung bezogen auf die Drehachse (80; 80") mindestens abschnittsweise in die Kupplungsausnehmung (62; 62") eingreift und dass das Instrumentarium eine Winkeleinstelleinrichtung (136") umfasst zum Einstellen einer Winkelstellung des Kopplungskörpers (24") und des Schablonenkörpers (26") bezogen auf die Drehachse (80") relativ zueinander.

11. Medizinisches Instrumentarium nach Anspruch 10, **dadurch gekennzeichnet, dass** die Winkeleinstelleinrichtung (136") eine Mehrzahl erster Winkeleinstellglieder (138") und mindestens ein zweites Winkeleinstellglied (140") umfasst und dass das mindestens eine zweite Winkeleinstellglied (140") in unterschiedlichen Winkelstellungen mit unterschiedlichen ersten Winkeleinstellgliedern (138") in Eingriff steht,
wobei weiter insbesondere die Mehrzahl erster Winkeleinstellglieder (138") in Form von Winkelgliedausnehmungen (142") ausgebildet sind und dass das mindestens eine zweite Winkeleinstellglied (140") in Form eines Winkelgliedvorsprungs (144") ausgebildet ist,
wobei
a) die Winkelgliedausnehmungen (142") am ersten oder zweiten Kupplungselement (42", 44") angeordnet oder ausgebildet sind und in Richtung auf die Drehachse (80") hin oder von der Drehachse (80") weg weisend angeordnet oder ausgebildet sind und/oder
b) der mindestens eine Winkelgliedvorsprung (144") federnd gelagert ist und beim Ändern der Winkelstellung durch Drehen der ersten und zweiten Kupplungselemente (42", 44") relativ zueinander um die Drehachse (80") entgegen einer vorspannenden Kraft aus einer der Winkelgliedausnehmungen (142") hinaus bewegt und in einer anderen Winkelstellung durch die vorspannende Kraft in eine der Winkelgliedausnehmungen (142") zurück bewegt wird.

12. Medizinisches Instrumentarium nach einem der voranstehenden Ansprüche, **dadurch gekennzeichnet, dass** der Kopplungskörper (24; 24") einen Griffbereich (56; 56") definiert,
wobei insbesondere der Kopplungskörper (24; 24") und der Schablonenkörper (26; 26'; 26") in der Kupplungsstellung relativ zueinander um eine durch die Kupplungseinrichtung (34; 34") definierte Drehachse (80; 80") verdrehbar aneinander gelagert sind und
a) der Griffbereich (56; 56") symmetrisch, insbesondere rotationssymmetrisch, bezogen auf die Drehachse (80; 80") ausgebildet ist und/oder
b) der mindestens eine Führungsschlitz (28; 28") eine Führungsebene (32) definiert, welche quer, insbesondere senkrecht, zur Drehachse (80; 80") verläuft.

13. Medizinisches Instrumentarium nach einem der voranstehenden Ansprüche, **dadurch gekennzeichnet, dass**
a) das Instrumentarium einen Raspelkörper (14) einer Prothesenraspel umfasst, welcher Raspelkörper (14) ein zweites Kopplungselement (20) einer das erste und das zweite Kopplungselement (18, 20; 18", 20) umfassenden Kopplungseinrichtung (22) umfasst, wobei insbesondere die ersten und zweiten Kopplungselemente (18, 20; 18", 20) in der Kopplungsstellung kraft- und/oder formschlüssig in Eingriff stehen und dass sie in einer Reinigungsstellung vollständig voneinander getrennt sind,
und/oder
b) das erste Kopplungselement (18; 18") in Form einer Kopplungselementaufnahme (82; 82") ausgebildet ist,
wobei insbesondere das zweite Kopplungselement (20) in Form eines zur Kopplungselementaufnahme (82; 82") korrespondierenden Kopplungsvorsprungs (86) ausgebildet ist.

14. Medizinisches Instrumentarium nach Anspruch 13, **dadurch gekennzeichnet, dass**
a) die Kopplungseinrichtung (22) eine Kopplungslängsachse (48; 48") definiert, welche insbesondere vom ersten und/oder zweiten Kopplungselement (18, 20; 18", 20) definiert ist,
wobei insbesondere die Kopplungslängsachse (48; 48") parallel oder im Wesentlichen parallel zur Drehachse (80; 80") verläuft und/oder die Drehachse (80; 80") die Kopplungslängsachse (48; 48") definiert,
und/oder
b) die ersten und zweiten Kopplungselemente (18, 20; 18", 20) in der Kopplungsstellung drehfest miteinander gekoppelt sind,
wobei insbesondere eines der beiden Rastglieder (110, 112; 110", 112') in Form eines Rastvorsprungs (108; 108") ausgebildet ist und dass das andere der beiden Rastglieder (110, 112; 110", 112') in Form einer korrespondierenden Rastausnehmung (102) ausgebildet ist,
wobei weiter insbesondere der Rastvorsprung (108; 108") derart ausgebildet ist, dass er beim in Eingriff Bringen des ersten Kopplungselements (18; 18") und des zweiten Kopplungselements (20) von einer Grundstellung in eine ausgelenkte Stellung ausgelenkt wird und erst dann, wenn die Kopplungseinrichtung (22) die Kopplungsstellung einnimmt, von der ausgelenkten Stellung in die Rastausnehmung (102) eingreifen kann, so dass die Sicherungseinrichtung (96) die Sicherungsstellung einnimmt.

15. Medizinisches Instrumentarium nach einem der voranstehenden Ansprüche, **dadurch gekennzeichnet, dass**
a) eines der beiden Sicherungselemente (98, 100; 98") am ersten Kopplungselement (18; 18") angeordnet oder ausgebildet ist und dass das andere der beiden Sicherungselemente (98, 100; 98") am zweiten Kopplungselement (20) angeordnet oder ausgebildet ist und/oder
b) die Sägeschablone (12; 12") zwei, drei, vier oder mehr Führungsschlitze (28; 28") umfasst, wobei die Führungsschlitze (28; 28") parallel oder im Wesentlichen parallel zueinander angeordnet oder ausgebildet sind.

## Claims

1. Medical instrumentarium (10; 10") for implanting a hip joint endoprosthesis, which instrumentarium (10; 10") comprises at least one sawing template (12; 12") with at least one guide slot (28; 28'; 28") for a saw blade (30), wherein the sawing template comprises at least one first coupling element (18; 18") for temporarily coupling the sawing template (12; 12") to a second coupling element (20) of a rasp body (14) of a prosthesis rasp in a coupling position, wherein a first securing element (98; 98") of a securing device (96) is associated with the first coupling element (18; 18"), which first securing element (98; 98") in the coupling position is in engagement in a force- and/or positive-locking manner in a securing position with a second securing element (100) arranged on the rasp body (14), **characterized in that** the first and the second securing element (98, 100; 98", 100) are configured in the form of cooperating latching members (110, 112).

2. Medical instrumentarium in accordance with Claim 1, **characterized in that** the sawing template (12; 12") comprises a coupling body (24; 24") and a template body (26; 26'; 26"), and **in that** the at least one first coupling element (18; 18") is arranged or formed on the coupling body (24; 24"), and **in that** the at least one guide slot (28; 28'; 28") is arranged or formed on the template body (26; 26'; 26").

3. Medical instrumentarium in accordance with Claim 2, **characterized by** a coupling device (34; 34") for temporarily coupling the coupling body (24; 24") and the template body (26; 26'; 26") in a coupling position.

4. Medical instrumentarium in accordance with Claim 3, **characterized in that**
a) the coupling device (34) is configured in the form of a latching or snapping connecting device (36) and comprises latching or snapping elements (38, 40) which are in engagement with each other in the coupling position
and/or
b) the coupling device (34; 34") comprises first and second coupling elements (42, 44; 42", 44"), **in that** the first and second coupling elements (42, 44; 42", 44") are arranged or formed on the coupling body (24; 24") on the one hand and on the template body (26; 26'; 26") on the other hand, and **in that** the first and second coupling elements (42, 44; 42", 44") in the coupling position are in engagement in a force- and/or positive-locking manner, wherein, in particular, the first and second coupling elements (42, 44) form the latching or snapping elements (38, 40),
and/or
c) the coupling body (24; 24") and the template body (26; 26'; 26") in the coupling position are mounted on each other so as to be rotatable relative to each other about a rotary axis (80; 80") defined by the coupling device (34; 34").

5. Medical instrumentarium in accordance with claim 3 or 4, **characterized in that** the coupling device (34; 34") comprises first and second coupling elements (42, 44; 42", 44"), **in that** the first and second coupling elements (42, 44; 42", 44") are arranged or formed on the coupling body (24; 24") on the one hand and on the template body (26; 26'; 26") on the other hand, and **in that** the first and second coupling elements (42, 44; 42", 44") in the coupling position are in engagement in a force- and/or positive-locking manner, **in that** the first and second coupling elements (42, 44; 42", 44") are configured in the form of at least one coupling projection (46; 46") and in the form of a corresponding coupling recess (62; 62").

6. Medical instrumentarium in accordance with Claim 5, **characterized in that**
a) the at least one coupling projection (46) is resiliently arranged or formed on the template body (26)
and/or
b) the at least one coupling projection (46) is configured in the form of a mounting bracket (50) which is concavely curved pointing toward the rotary axis
and/or
c) the at least one coupling recess (62; 62") is configured in the form of an annular groove (64; 64") which is open pointing away from the rotary axis (80; 80"), and **in that** the at least one coupling projection in the coupling position engages at least in sections into the coupling recess (62; 62") in circumferential direction in relation to the rotary axis (80; 80").

7. Medical instrumentarium in accordance with claim 5 or 6, **characterized in that** the coupling body (24; 24") and the template body (26; 26'; 26") in the coupling position are mounted on each other so as to be rotatable relative to each other about a rotary axis (80; 80") defined by the coupling device (34; 34"), and **in that** the at least one coupling projection (46") is configured in the form of a coupling ring (132") which is closed in itself or extends over at least 180° in circumferential direction in relation to the rotary axis (80").

8. Medical instrumentarium in accordance with any one of Claims 5 to 7, **characterized in that** the at least one coupling recess (62; 62") is configured in the form of an annular groove (64; 64") which is open pointing away from the rotary axis (80; 80"), and **in that** the at least one coupling projection in the coupling position engages at least in sections into the coupling recess (62; 62") in circumferential direction in relation to the rotary axis (80; 80"), and **in that** the annular groove (64") is delimited laterally by a retaining ring (128"),
wherein, in particular, the retaining ring (128") and the coupling body (24") are connected to each other in a force- and/or positive-locking manner and/or by material bond.

9. Medical instrumentarium in accordance with any one of Claims 5 to 8, **characterized in that** the at least one coupling recess (62; 62") is configured in the form of an annular groove (64; 64") which is open pointing away from the rotary axis (80; 80"), and **in that** the at least one coupling projection in the coupling position engages at least in sections into the coupling recess (62; 62") in circumferential direction in relation to the rotary axis (80; 80"), and **in that** the first and second coupling elements (42", 44") are alignable relative to each other in discrete angular positions in relation to the rotary axis (80").

10. Medical instrumentarium in accordance with any one of Claims 5 to 9, **characterized in that** the at least one coupling recess (62; 62") is configured in the form of an annular groove (64; 64") which is open pointing away from the rotary axis (80; 80"), and **in that** the at least one coupling projection in the coupling position engages at least in sections into the coupling recess (62; 62") in circumferential direction in relation to the rotary axis (80; 80"), and **in that** the instrumentarium comprises an angle adjusting device (136") for adjusting an angular position of the coupling body (24") and the template body (26") in relation to the rotary axis (80") relative to each other.

11. Medical instrumentarium in accordance with Claim 10, **characterized in that** the angle adjusting device (136") comprises a plurality of first angle adjusting members (138") and at least one second angle adjusting member (140"), and **in that** the at least one second angle adjusting member (140") is in engagement with different first angle adjusting members (138") in different angular positions,
wherein, further in particular, the plurality of first angle adjusting members (138") are configured in the form of angle member recesses (142"), and **in that** the at least one second angle adjusting member (140") is configured in the form of an angle member projection (144"), wherein
a) the angle member recesses (142") are arranged or formed on the first or second coupling element (42", 44") and are arranged or formed pointing in the direction toward the rotary axis (80") or away from the rotary axis (80")
and/or
b) the at least one angle member projection (144") is resiliently mounted and, when changing the angular position by rotating the first and second coupling elements (42", 44") relative to each other about the rotary axis (80"), is moved out of one of the angle member recesses (142") counter to a biasing force and in another angular position is moved back into one of the angle member recesses (142") as a result of the biasing force.

12. Medical instrumentarium in accordance with any one of the preceding Claims, **characterized in that** the coupling body (24; 24") defines a grip region (56; 56"),
wherein, in particular, the coupling body (24; 24") and the template body (26; 26'; 26") in the coupling position are mounted on each other so as to be rotatable relative to each other about a rotary axis (80; 80") defined by the coupling device (34; 34"), and
a) the grip region (56, 56") is formed symmetrically, in particular rotationally symmetrically, in relation to the rotary axis (80; 80") and/or
b) the at least one guide slot (28; 28") defines a guidance plane (32) which runs transversely, in particular perpendicularly, to the rotary axis (80; 80").

13. Medical instrumentarium in accordance with any one of the preceding Claims, **characterized in that**
a) the instrumentarium comprises a rasp body (14) of a prosthesis rasp, which rasp body (14) comprises a second coupling element (20) of a coupling device (22) comprising the first and the second coupling element (18, 20; 18", 20),
wherein, in particular, the first and second coupling elements (18, 20; 18", 20) are in engagement with each other in a force- and/or positive-locking manner in the coupling position, and **in that** they are completely separated from each other in a cleaning position, and/or
b) the first coupling element (18; 18") is configured in the form of a coupling element receptacle (82; 82"),
wherein, in particular, the second coupling element (20) is configured in the form of a coupling projection (86) corresponding to the coupling element receptacle (82; 82").

14. Medical instrumentarium in accordance with Claim 13, **characterized in that**
a) the coupling device (22) defines a coupling longitudinal axis (48; 48") which is defined in particular by the first and/or second coupling element (18, 20; 18", 20),
wherein, in particular, the coupling longitudinal axis (48; 48") runs parallel or substantially parallel to the rotary axis (80; 80") and/or in that the rotary axis (80; 80") defines the coupling longitudinal axis (48; 48"),
and/or
b) the first and second coupling elements (18, 20; 18", 20) are non-rotatably coupled to each other in the coupling position
wherein, in particular, one of the two latching members (110, 112; 110", 112') is configured in the form of a latching projection (108; 108"), and **in that** the other one of the two latching members (110, 112; 110", 112') is configured in the form of a corresponding latching recess (102),
wherein, further in particular, the latching projection (108; 108") is configured in such a way that, when bringing the first coupling element (18; 18") and the second coupling element (20) into engagement, it is deflected from a base position into a deflected position, and only when the coupling device (22) adopts the coupling position is it able to engage into the latching recess (102) from the deflected position, such that the securing device (96) adopts the securing position.

15. Medical instrumentarium in accordance with any one of the preceding Claims, **characterized in that**
a) one of the two securing elements (98, 100; 98") is arranged or formed on the first coupling element (18; 18"), and **in that** the other one of the two securing elements (98, 100; 98") is arranged or formed on the second coupling element (20)
and/or
b) the sawing template (12; 12") comprises two, three, four or more guide slots (28; 28"), wherein the guide slots (28; 28") are arranged or formed in parallel or substantially in parallel to each other.

## Revendications

1. Ensemble d'instruments médicaux (10; 10") pour l'implantation d'une endoprothèse de l'articulation de la hanche, lequel ensemble d'instruments médicaux (10; 10") comprend au moins un gabarit de scie (12; 12") avec au moins une fente de guidage (28; 28'; 28") pour une lame de scie (30), où le gabarit de scie comprend au moins un premier élément de couplage (18; 18") pour le couplage temporaire du gabarit de scie (12; 12") à un deuxième élément de couplage (20) d'un corps de râpe (14) d'une râpe de prothèse dans une position de couplage, où au premier élément de couplage (18; 18") est associé un premier élément de fixation (98; 98") d'un dispositif de fixation (96), qui, dans la position de couplage, est en prise par assemblage de force et/ou de forme avec un deuxième élément de fixation (100) agencé sur le corps de râpe (14) dans une position de fixation, **caractérisé en ce que** le premier et le deuxième élément de fixation (98, 100; 98", 100) sont conçus sous forme de membres d'encliquetage (110, 112) qui coopèrent.

2. Ensemble d'instruments médicaux selon la revendication 1, **caractérisé en ce que** le gabarit de scie (12; 12") comprend un corps de couplage (24; 24") et un corps de gabarit (26; 26'; 26") et **en ce que** le au moins un premier élément de couplage (18; 18") est agencé ou formé sur le corps de couplage (24; 24") et **en ce que** la au moins une fente de guidage (28; 28'; 28") est agencée ou formée sur le corps de gabarit (26; 26'; 26").

3. Ensemble d'instruments médicaux selon la revendication 2, **caractérisé par** un dispositif de couplage (34; 34") pour le couplage temporaire du corps de couplage (24; 24") et du corps de gabarit (26; 26'; 26") dans une position de couplage.

4. Ensemble d'instruments médicaux selon la revendication 3, **caractérisé en ce que**
a) le dispositif de couplage (34) est formé sous la forme d'un dispositif de liaison par encliquetage ou emboîtement (36) et comprend des éléments d'encliquetage ou d'emboîtement (38, 40) qui sont en prise l'un avec l'autre dans la position de couplage
et/ou
b) le dispositif de couplage (34; 34") comprend des premier et deuxième éléments de couplage (42, 44; 42", 44"), **en ce que** les premier et deuxième éléments de couplage (42, 44; 42", 44") sont agencés ou formés d'une part sur le corps de couplage (24; 24") et d'autre part sur le corps de gabarit (26; 26'; 26") et **en ce que** les premier et deuxième éléments de couplage (42, 44; 42", 44") sont en prise par assemblage de force et/ou de forme dans la position de couplage,
où en particulier les premier et deuxième éléments de couplage (42, 44) forment les éléments d'encliquetage ou d'emboîtement (38, 40),
et/ou
c) le corps de couplage (24; 24") et le corps de gabarit (26; 26'; 26") sont montés l'un contre l'autre de manière à pouvoir tourner l'un par rapport à l'autre dans la position de couplage autour d'un axe de rotation (80; 80") défini par le dispositif de couplage (34; 34").

5. Ensemble d'instruments médicaux selon la revendication 3 ou 4, **caractérisé en ce que** le dispositif de couplage (34; 34") comprend des premier et deuxième éléments de couplage (42, 44; 42", 44"), **en ce que** les premier et deuxième éléments de couplage (42, 44; 42", 44") sont agencés ou formés d'une part sur le corps de couplage (24; 24") et d'autre part sur le corps de gabarit (26; 26'; 26") et **en ce que** les premier et deuxième éléments de couplage (42, 44; 42", 44") sont en prise par assemblage de force et/ou de forme dans la position de couplage, **en ce que** les premier et deuxième éléments de couplage (42, 44; 42", 44") sont formés sous la forme d'au moins une saillie de couplage (46; 46") et sous la forme d'un évidement de couplage (62; 62") correspondant.

6. Ensemble d'instruments médicaux selon la revendication 5, **caractérisé en ce que**
a) la au moins une saillie de couplage (46) est agencée ou formée élastiquement sur le corps de gabarit (26)
et/ou
b) la au moins une saillie de couplage (46) est formée sous la forme d'un étrier de retenue (50) courbé de manière concave en étant orienté vers l'axe de rotation
et/ou
c) le au moins un évidement de couplage (62; 62") est formé sous la forme d'une rainure annulaire ouverte (64; 64") orientée à l'opposé de l'axe de rotation (80; 80") et **en ce que** la au moins une saillie de couplage s'engage au moins par sections dans l'évidement de couplage (62; 62") dans la position de couplage dans la direction de la circonférence par rapport à l'axe de rotation (80; 80").

7. Ensemble d'instruments médicaux selon la revendication 5 ou 6, **caractérisé en ce que** le corps de couplage (24; 24") et le corps de gabarit (26; 26'; 26") sont montés l'un contre l'autre de manière à pouvoir tourner, dans la position de couplage, l'un par rapport à l'autre autour d'un axe de rotation (80; 80") défini par le dispositif de couplage (34; 34") et **en ce que** la au moins une saillie de couplage (46") est formée sous la forme d'une bague de couplage (132") fermée sur elle-même ou s'étendant sur au moins 180° dans la direction de la circonférence par rapport à l'axe de rotation (80").

8. Ensemble d'instruments médicaux selon l'une des revendications 5 à 7, **caractérisé en ce que** le au moins un évidement de couplage (62; 62") est formé sous la forme d'une rainure annulaire ouverte (64; 64") orientée à l'opposé de l'axe de rotation (80; 80") et **en ce que**, dans la position de couplage, la au moins une saillie de couplage s'engage au moins par sections dans l'évidement de couplage (62; 62") dans la direction de la circonférence par rapport à l'axe de rotation (80; 80") et **en ce que** la rainure annulaire (64") est limitée latéralement par une bague de retenue (128"),
où en particulier la bague de retenue (128") et le corps de couplage (24") sont reliés l'un à l'autre par assemblage de force et/ou de forme et/ou de matière.

9. Ensemble d'instruments médicaux selon l'une des revendications 5 à 8, **caractérisé en ce que** le au moins un évidement de couplage (62; 62") est formé sous la forme d'une rainure annulaire ouverte (64; 64") orientée à l'opposé de l'axe de rotation (80; 80") et **en ce que**, dans la position de couplage, la au moins une saillie de couplage s'engage au moins par sections dans l'évidement de couplage (62; 62" ) dans la direction de la circonférence par rapport à l'axe de rotation (80; 80") et **en ce que** les premier et deuxième éléments de couplage (42", 44") peuvent être orientés l'un par rapport à l'autre dans des positions angulaires discrètes par rapport à l'axe de rotation (80").

10. Ensemble d'instruments médicaux selon l'une des revendications 5 à 9, **caractérisé en ce que** le au moins un évidement de couplage (62; 62") est formé sous la forme d'une rainure annulaire ouverte (64; 64") orientée à l'opposé de l'axe de rotation (80; 80") et **en ce que** la au moins une saillie de couplage, dans la position de couplage, s'engage au moins par sections dans l'évidement de couplage (62; 62") dans la direction de la circonférence par rapport à l'axe de rotation (80; 80") et **en ce que** l'ensemble d'instruments comprend un dispositif de réglage d'angle (136") pour régler une position angulaire du corps de couplage (24") et du corps de gabarit (26") l'un par rapport à l'autre par rapport à l'axe de rotation (80").

11. Ensemble d'instruments médicaux selon la revendication 10, **caractérisé en ce que** le dispositif de réglage d'angle (136") comprend une pluralité de premiers membres de réglage d'angle (138") et au moins un deuxième membre de réglage d'angle (140") et **en ce que** le au moins un deuxième membre de réglage d'angle (140") est en prise dans différentes positions angulaires avec différents premiers membres de réglage d'angle (138"),
où en outre en particulier la pluralité de premiers membres de réglage d'angle (138") sont formés sous la forme d'évidements de membre d'angle (142") et **en ce que** le au moins un deuxième membre de réglage d'angle (140") est formé sous forme d'une saillie de membre d'angle (144"),
où
a) les évidements de membre d'angle (142") sont agencés ou formés sur le premier ou le deuxième élément de couplage (42", 44") et agencés ou formés en direction de l'axe de rotation (80") ou en étant orientés à l'opposé de l'axe de rotation (80")
et/ou
b) la au moins une saillie de membre d'angle (144") est montée élastiquement et lors du changement de la position angulaire par rotation des premier et deuxième éléments de couplage (42", 44") l'un par rapport à l'autre autour de l'axe de rotation (80") est déplacée contre une force de sollicitation hors de l'un des évidements de membre d'angle (142") et ramenée dans l'un des évidements de membre d'angle (142") dans une autre position angulaire par la force de sollicitation.

12. Ensemble d'instruments médicaux selon l'une des revendications précédentes, **caractérisé en ce que** le corps de couplage (24; 24") définit une zone de préhension (56; 56"),
où en particulier le corps de couplage (24; 24") et le corps de gabarit (26; 26'; 26") sont montés l'un contre l'autre de manière à pouvoir tourner l'un par rapport à l'autre dans la position de couplage autour d'un axe de rotation (80; 80") défini par le dispositif de couplage (34; 34") et
a) la zone de préhension (56; 56") est formée de manière symétrique, en particulier de manière symétrique en rotation, par rapport à l'axe de rotation (80; 80")
et/ou
b) la au moins une fente de guidage (28; 28") définit un plan de guidage (32) qui s'étend transversalement, en particulier perpendiculairement, à l'axe de rotation (80; 80").

13. Ensemble d'instruments médicaux selon l'une des revendications précédentes, **caractérisé en ce que**
a) l'ensemble d'instruments comprend un corps de râpe (14) d'une râpe de prothèse, lequel corps de râpe (14) comprend un deuxième élément de couplage (20) d'un dispositif de couplage (22) comprenant le premier et le deuxième élément de couplage (18, 20; 18", 20),
où en particulier les premier et deuxième éléments de couplage (18, 20; 18", 20) sont en prise, dans la position de couplage, par assemblage de force et/ou de forme et **en ce qu'**ils sont totalement séparés l'un de l'autre dans une position de nettoyage,
et/ou
b) le premier élément de couplage (18; 18") est formé sous la forme d'un logement d'élément de couplage (82; 82"),
où en particulier le deuxième élément de couplage (20) est formé sous la forme d'une saillie de couplage (86) correspondant au logement d'élément de couplage (82; 82").

14. Ensemble d'instruments médicaux selon la revendication 13, **caractérisé en ce que**
a) le dispositif de couplage (22) définit un axe longitudinal de couplage (48; 48") qui est défini en particulier par le premier et/ou le deuxième élément de couplage (18, 20; 18", 20),
où en particulier l'axe longitudinal de couplage (48; 48") s'étend parallèlement ou sensiblement parallèlement à l'axe de rotation (80; 80") et/ou l'axe de rotation (80; 80") définit l'axe longitudinal de couplage (48; 48"),
et/ou
b) les premier et deuxième éléments de couplage (18, 20; 18", 20) sont couplés l'un à l'autre de manière solidaire en rotation dans la position de couplage,
où en particulier l'un des deux membres d'encliquetage (110, 112; 110", 112') est formé sous la forme d'une saillie d'encliquetage (108; 108") et **en ce que** l'autre des deux membres d'encliquetage (110, 112; 110", 112') est formé sous forme d'un évidement d'encliquetage correspondant (102),
où en outre en particulier la saillie d'encliquetage (108; 108") est formée de telle manière qu'elle est déviée d'une position de base à une position déviée lors de la mise en prise du premier élément de couplage (18; 18") et du deuxième élément de couplage (20) et ne peut s'engager dans l'évidement d'encliquetage (102) à partir de la position déviée que lorsque le dispositif de couplage (22) prend la position de couplage, de sorte que le dispositif de fixation (96) prend la position de fixation.

15. Ensemble d'instruments médicaux selon l'une des revendications précédentes, **caractérisé en ce que**
a) l'un des deux éléments de fixation (98, 100; 98") est agencé ou formé sur le premier élément de couplage (18; 18") et **en ce que** l'autre des deux éléments de fixation (98, 100; 98") est agencé ou formé sur le deuxième élément de couplage (20)
et/ou
b) le gabarit de scie (12; 12") comprend deux, trois, quatre fentes de guidage (28; 28") ou plus, où les fentes de guidage (28; 28") sont agencées ou formées parallèlement ou sensiblement parallèlement entre elles.
